# EUROPEAN PATENT APPLICATION

(11) **EP 1 865 324 A1**
(43) Date of publication of application: **12.12.2007**
(21) Application number: 07106904.1
(22) Date of filing: 25.04.2007
(51) Int. Cl.: G01N 33/543

(54) **Diagnostic methods using magnetic nanoparticles**

(30) Priority: 06.06.2006 US 422336
(71) Applicant: Hong, Chin-Yih Rex, 104 Taipei (TW); Horng, Herng-Er, 104 Taipei (TW); Wu, Chau-Chung, Jhongjheng District 100 Taipei (TW); Yang, Hong-Chang, 104 Taipei (TW); Yang, Shieh-Yueh, Sindian City, 231 Taipei County (TW)
(72) Inventor: Hong, Chin-Yih Rex, 104 Taipei (TW); Horng, Herng-Er, 104 Taipei (TW); Wu, Chau-Chung, Jhongjheng District 100 Taipei (TW); Yang, Hong-Chang, 104 Taipei (TW); Yang, Shieh-Yueh, Sindian City, 231 Taipei County (TW)
(74) Representative: Reichert, Werner Franz

(57) **Abstract**

The present invention is designed as the diagnostic methods using magnetic nanoparticles to quantitatively measure the ligands or biomolecules for assessing/evaluating the status or risks of diseases, such as atherosclerosis, infection/inflammatory diseases, and tumors. Through the use of the magnetic nanoparticles and the bio-receptors coated to the magnetic nanoparticles, the ligands conjugated with the bio-receptors can be detected or marked, and the amount of the ligands in a sample can be determined by measuring the changes in magnetic properties resulting from the existence of the ligands.

## Description

The present invention generally relates to a method for measuring biomolecules/ligands. In particular, the present invention relates to diagnostic methods using magnetic labelling immunoassays with magnetic nanoparticles.

Nowadays, it is common to use biomolecules (molecular biomarkers) associated with disease processes for evaluation, assessment or even diagnosis of the diseases. These biomarkers may be molecules or factors predisposing to the diseases or the occurrence of cell-surface markers, enzymes or other components. Especially for vital diseases or distressing symptoms, the related biomarkers can be very informative for early identification or better treatments.

Cardiovascular diseases, including atherosclerosis, are leading diseases of death for both men and women among most ethnic groups. Atherosclerosis always accompanies with vulnerable plaques, especially unstable atherosclerotic plaques (UAPs). UAPs frequently express proteins such as, vascular cell adhesion molecule-1 (VCAM-1), matrix metalloproteinase (MMP), intracellular adhesion molecule-1 (ICAM-1) and vascular endothelial growth factor (VEGF). In addition, recent medical reports show that atherosclerosis leads to a high-level high-sensitive C-reactive protein (hsCRP). Hence, the detection of these proteins can help identify the existence of UAPs for the risk assessments of atherosclerosis.

Nevertheless, the CRP level is not only an indication to the risk assessment of atherosclerotic, but also a key indicator of infectious/inflammatory diseases. When tissues are damaged during the course of infectious/inflammatory diseases, cytokine is produced and induces liver to produce CRP and pigment epithelium-derived factor (PEDF). Because the CRP or PEDF levels increase dramatically in the event of injury or infection, the CRP or PEDF levels have become key indicators of infectious/inflammatory diseases. Moreover, because VEGF is closely related to the growth of tumors, the VEGF level can be used as an indicator for the risk assessment of tumors.

As nanotechnology advances rapidly, further biological or medical applications of nanoparticles have been investigated. It has been proposed that magnetic nanoparticles can be used for labelling biomolecules or biological targets. At present, the magnetic nanoparticles need to be applied along with some optical or coloring agents, so that the biological targets labelled with these nanoparticles can be detected. However, further processing steps or preparation procedures are required for linking the optical or coloring agents, and extra manual labor and costs are needed for the application of molecular.

Accordingly, the present invention is directed to diagnostic methods using magnetic nanoparticles to quantitatively measure the ligands for assessing/evaluating the status or risks of diseases. The methods provided by this invention can also be applied to measure biomolecules for analytical purposes.

The present invention is directed to diagnostic methods for quantitatively measuring the ligands or biomolecules by using magnetic labelling immunoassays with magnetic nanoparticles. Through the use of these magnetic nanoparticles and the bio-receptors coated to the magnetic nanoparticles, the ligands conjugated with the bio-receptors result in the formation of particle clusters. The differences between magnetic properties of free magnetic nanoparticles and the formed particle clusters can be measured for determining the amount of the ligands. For example, regarding the diagnostic methods for atherosclerosis, infection/inflammatory diseases and tumors, the ligands can be vascular cell adhesion molecule-1 (VCAM-1), matrix metalloproteinase (MMP), intracellular adhesion molecule-1 (ICAM-1), vascular endothelial growth factor (VEGF), C-reactive protein (CRP), high-sensitive C-reactive protein (hsCRP), or pigment epithelium-derived factor (PEDF).

According to one embodiment of the present invention, a diagnostic method using a magnetic labelling immunoassay for in-vitro quantitatively measuring the amount of ligands in a sample solution is proposed, comprising: providing the sample solution containing the ligands, applying the magnetic labelling immunoassay to the sample solution, filtrating the sample solution to obtain the particle clusters, and measuring a saturated magnetization of the particle clusters to determine the amount of the ligands.

According to another embodiment of the present invention, a diagnostic method using a magnetic labelling immunoassay for in-vitro quantitatively measuring an amount of ligands in a sample solution is proposed, comprising: providing the sample solution containing the ligands, applying the magnetic labelling immunoassay to the sample solution, and measuring an ac magnetic susceptibility reduction of the sample solution to determine the amount of the ligands.

The present invention also relates to a magnetic labelling immunoassay to detect ligands in a sample for assessing/evaluating statuses or risks of diseases, comprising: magnetic nanoparticles, hydrophilic surfactants coated on surfaces of the magnetic nanoparticles; and bio-receptors bound to the hydrophilic surfactants on the magnetic nanoparticles. The bio-receptors in the immunoassay are able to conjugate with the ligands in the sample.

Because the measuring methods proposed in this invention are performed by measuring magnetic properties of the magnetic nanoparticles and/or the formed particle clusters, no fluorescence labels or coloring agents are required for determining the amount of the ligands in the sample. Hence, no extra processing steps and less human labor are needed and the costs of the test assays can be reduced.

The accompanying drawings are included to provide a further understanding of the invention, and are incorporated in and constitute a part of this specification. The drawings illustrate embodiments of the invention and, together with the description, serve to explain the principles of the invention.

Fig. 1 shows the relationship between the optical density and various amounts of HUVECs (the ELISA curve).

Fig. 2 is a schematic model for the immunomagnetic labelling of VCAM-1 on HUVECs.

Fig. 3 shows the relationship of the saturated magnetization Mₛ of the magnetic labelled VCAM-1 on HUVECs and various amounts of HUVECs (the MLI curve), in comparison with the ELISA curve shown in Fig. 1.

Fig. 4 shows the curves of the saturated magnetization Mₛ or the optical density of the bound magnetic nanoparticles vs the concentration φ of human CRP.

Fig. 5 shows the saturated magnetization difference (Mₛ - M_{s,o}) of the magnetic nanoparticles with CRP immune complexes as a function of CRP concentrations φ.

Fig. 6 shows the relationship of the normalized ac magnetic susceptibility reductions (Δχ_{ac}/χ_{ac,o}) and the amounts of VEGF using variously concentrated (Mₛ) magnetic fluids.

Reference will now be made in detail to the present preferred embodiments of the invention, examples of which are illustrated in the accompanying drawings. Wherever possible, the same reference numbers are used in the drawings and the description to refer to the same or like parts.

The present invention proposes a method of quantitatively measuring ligands or biomolecules in a sample by using magnetic nanoparticles based on the saturated magnetization or the alternating current (ac) magnetic susceptibility reduction. The details of the above mechanisms are described in the prior US Patent application 11/164275.

For the method based on saturation magnetization, it can be principally classified as the following steps: providing a solution having magnetic nanoparticles; coating bio-receptors to the surfaces of the magnetic nanoparticles; adding the solution to the sample containing ligands or biomolecules to be detected, so that the ligands or biomolecules in the sample conjugate with the bio-receptors and the nanoparticles agglomerate to form particle clusters; filtrating the solution to obtain the particle clusters; and measuring the saturated magnetization of the particle clusters to obtain the amount of the ligands or biomolecules.

For the method based on the alternating current (ac) magnetic susceptibility reduction, it can be principally classified as the following steps: providing a solution having magnetic nanoparticles; coating bio-receptors to the surfaces of the magnetic nanoparticles in the solution; measuring the ac magnetic susceptibility of the solution before and after adding a sample containing the ligands or biomolecules to be detected to the solution, so as to obtain an ac magnetic susceptibility reduction or a normalized ac magnetic susceptibility to determine the amount of the ligands or biomolecules.

Certain aspects of the above steps will be explained in more details in the following paragraphs.

### I. Preparation of bio-functionalized magnetic nanoparticles

*Preparation of magnetic nanoparticles containing solution.* Herein, Fe₃O₄ nanoparticle is used as an example of the magnetic nanoparticle for the present invention; however, other possible magnetic nanoparticles, including MnFe₂O₄, CoFe₂O₄, NiFe₂O₄, or Fe₂O₃, may also be utilized and be comprised within the scope of this invention. A ferrite solution containing Fe²⁺ and Fe³⁺ in 1:2 stoichiometric ratio (molar ratio), was mixed with water containing polar molecules. The polar molecule acts as a surfactant for helping dispersing the Fe₃O₄ particles in water or alternatively for improving binding of the bio-receptors to the surface of the Fe₃O₄ particles. For example, the surfactant can be dextran. However, other possible surfactant may also be utilized and be comprised within the scope of this invention. Hydroxide ions (OH-) were then added to the mixture for adjusting the pH value to around 8-11 to form black Fe₃O₄ nanoparticles. Aggregates and excess unbound surfactants were removed and the obtained solution comprising Fe₃O₄ nanoparticles can be referred as the magnetic fluid. The hydrodynamic diameter of the Fe₃O₄ particles was controlled to be between 25 nm to 90 nm by adjusting the initial pH value or other parameters.

*Binding of bio-receptors onto magnetic nanoparticles.* Then, bio-receptors were added to the solution and bound with the oxidized surfactants on the surface of the Fe₃O₄ particles, so as to prepare the Fe₃O₄ particles coated with the bio-receptors. Afterwards, the bio-functionalized (i.e. coated with the bio-receptors) magnetic nanoparticles are collected through magnetic separation, and then re-dissolved into a phosphate-buffered saline (PBS) solution. Hence, the solution containing magnetic particles coated with the bio-receptors is obtained.

Afterwards, the solution that contains magnetic particles coated with the bio-receptors is used for detecting the conjugated ligands or measuring the amount of ligands existing in a sample to be tested, by adding the sample to the solution. The choice of the used bio-receptors may vary depending on the ligands to be detected. According to this invention, the bio-receptors will bind or conjugate with the ligands to be detected. Because the bio-receptors conjugate with ligands to be detected, the Fe₃O₄ particles may aggregate as clusters through the conjugation of bio-receptors-and-ligands, especially if the single bio-receptor can conjugate with multiple ligands. In this embodiment, for example, some of the bio-receptors can be modified with streptavidin-biotin pair (i.e. streptavidin-biotinylated) for enhancing the affinity toward the conjugated ligands.

In Table I, examples of possible ligands and corresponding bio-receptors are lists for the magnetic Fe₃O₄ nanoparticles. However, a variety of ligands or biomolecules and corresponding conjugates thereof can be used in this invention as long as suitable affinity may be established between the conjugated or binding pair, and the scope of this invention will not be limited by the listed examples. For example, the biomolecule to be tested or measured may be a protein, polysaccharides, a lipoprotein or a glycoprotein, while the bio-receptor can be corresponding monoclonal or polyclonal antibodies, biotinylated antibodies or their natural/artificial conjugates. Examples of the conjugated pair (ligands and corresponding bio-receptors) are listed in Table 1. Potential applications of the conjugated pair (ligands and corresponding bio-receptors) include diagnosis, identification, or cure of atherosclerosis, tumor, cancer, acute injury, infections, or inflammatory diseases. It should be noted that the either one of the conjugated pair (ligands and bio-receptors) listed in the table can be coated onto the surface of the magnetic nanoparticles for detecting the other of the conjugated pair.

**Table 1. Examples of potential ligands and corresponding bio-receptors for the magnetic Fe₃O₄ nanoparticles**

| Ligand | Bio-receptor |
|---|---|
| Vascular cell adhesion molecule-1 (VCAM-1) | Streptavidin-biotinylated anti-VCAM-1 |
| Matrix metalloproteinase (MMP) | Streptavidin-biotinylated anti-MMP |
| Intracellular adhesion molecule-1 (ICAM-1) | Streptavidin-biotinylated anti-ICAM-1 |
| Vascular endothelial growth factor (VEGF) | Streptavidin-biotinylated anti-VEGF |
| C-reactive protein (CRP) | Anti-CRP |
| High-sensitivity CRP (hsCRP) | Anti-CRP |
| Pigment epithelium-derived factor (PEDF) | Streptavidin-biotinylated anti-PEDF |

### II. Detection of ligands labelled with bio-functionalized magnetic nanoparticles

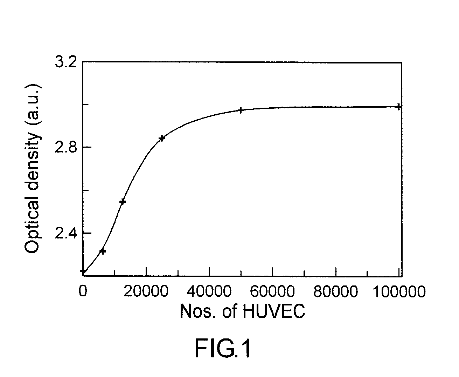

*ELISA detection of VCAM-1 on cells.* As a layer of collagen is spread onto a membrane, human umbilicalvan endothelic cells (HUVEC) (obtained National Taiwan University Hospital) are then transferred onto the collagen layer for incubation, followed by adding tumor necrosis factor-α (TNF-α) to inflammatorily stimulate HUVECs to release VCAM-1. Several hours later, methanol is used to stop the release of VCAM-1. The traditional enzyme-linked immunosorbent assay (ELISA) is performed to verify the existence of VCAM-1. Various amounts of HUVECs, which correspondingly generate various amounts of VCAM-1 after stimulation, are used in the incubation, while the released VCAM-1 molecules are detected by ELISA shown as the optical density. Fig. 1 shows the relationship between the optical density and various amounts of HUVECs (the ELISA curve).

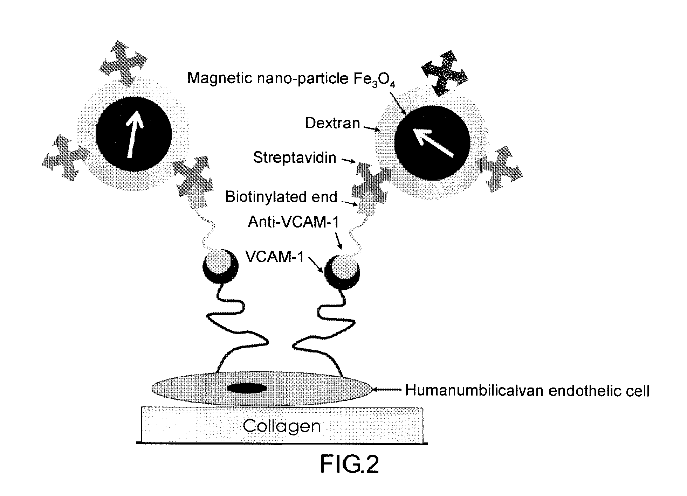

*Immunomagnetic detection of magnetically labelled VCAM-1 on HUVECs.* By using the magnetic nanoparticles bio-functionalized with anti-VCAM-1, the VCAM-1 on the incubated HUVECs can be magnetically labelled and/or detected, as schematically shown in Fig. 2. In this experiment, exceed amounts of bio-functionalized (i.e. modified with streptavidin-biotinylated anti-VCAM-1) magnetic nanoparticles are added to the samples incubated with various numbers of HUVECs, and PBS solution is then used to wash away exceed magnetic nanoparticles from the samples. Various amounts of HUVECs, which correspondingly generate various amounts of VCAM-1 after stimulation, are used in the incubation, while the released VCAM-1 molecules are detected by the bio-functionalized magnetic nanoparticles. Then, the magnetic properties of these magnetically labelled VCAM-1-incubated HUVECs are characterized.

The detailed mechanisms of magnetic detection based on the saturated magnetization or the alternating current (ac) magnetic susceptibility reduction are discussed in the prior US Patent application 11/164275, and will not be described in details herein.

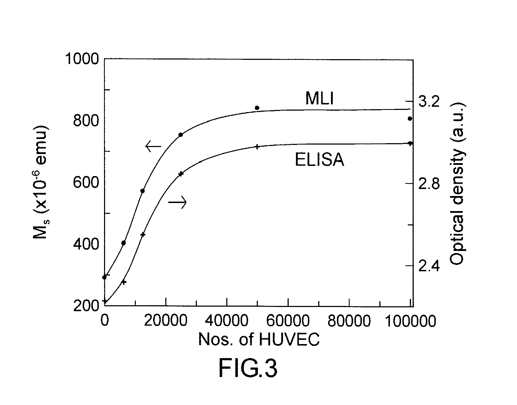

Saturated magnetizations Mₛ of the samples with various amounts of HUVECs are measured using a superconducting quantum interference device (SQUID) gradiometer. Fig. 3 shows the relationship of the saturated magnetization Mₛ of the magnetic labelled VCAM-1 on HUVECs and various amounts of HUVECs (the MLI curve), in comparison with the ELISA curve shown in Fig. 1. That is, saturated magnetization Mₛ of the magnetic nanoparticles bound to VCAM-1 on HUVEC is plotted as a function of various amounts of HUVEC to obtain the MLI curve. From Fig. 3, it clearly shows that the MLI curve is highly agreeable with the ELISA curve. This verifies the validity of detecting ligands (e.g. VCAM-1) using the bio-functionalized (e.g. anti-VCAM-1-functionalized) magnetic nanoparticles. The MLI curve in Fig. 3 provides a reference relationship between the amount of the ligands and the saturated magnetization of the bound bio-functionalized magnetic nanoparticles to quantitatively detect the ligand VCAM-1.

*Immunomagnetic detection of CRP.* Magnetic nanoparticles bio-functionalized with anti-CRP can be used to assay human CRP. When CRP was bound to the magnetic nanoparticles, the mean hydrodynamic diameter of the bound magnetic nanoparticles became larger due to the formation of the immune complex (CRP-anti-CRP-dextran-magnetic nanoparticles). For example, when 0.1 ml of CRP solution (obtained from Sigma-Aldrich Inc., containing 12 ng human CRP) was mixed with 20-µl of the magnetic fluid (bio-functionalized with anti-CRP), the mean hydrodynamic diameter of the bound magnetic nanoparticles increased from 46.1 nm to 85.7 nm. Through filtration using a filter with micro-holes of 50 nm in diameter, the immune complexes are separated from the solution and stay on the filter. The saturated magnetization Mₛ of the bound magnetic nanoparticles staying on the filter was measured with a SQUID gradiometer system.

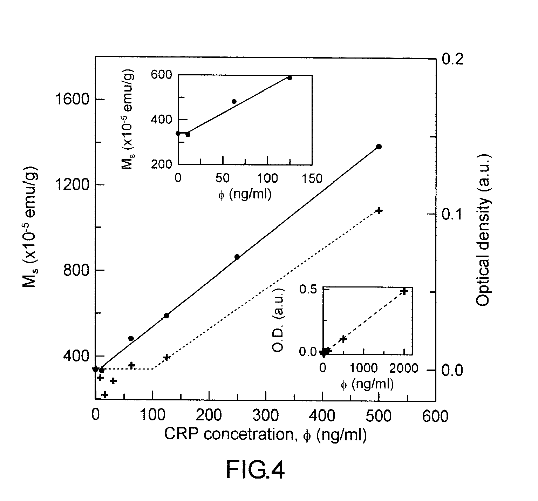

Fig. 4 shows the curves of the saturated magnetization Mₛ or the optical density of the bound magnetic nanoparticles vs. the concentration φ of human CRP. The solidline curve in Fig. 4 represents the relationship between Mₛ of the immune complexes and the concentration φ of human CRP, and the mathematical formula of the Mₛ-φ curve (solid line curve) is as follows: Mₛ = 2.1 x 10⁻⁵ φ + 0.0033, for φ ≥ 10 ng/ml. For φ < 10 ng/ml, the value of Mₛ remained unchanged, as shown in the upper-left inset of Fig. 4. This indicates that the sensitivity of magnetically labelled immunoassay for human CRP via saturated magnetization measurement is 10 ng/ml or 1 ng in the unit of mass. On the other hand, the ELISA detection results shown as the optical density (OD) of the bound magnetic nanoparticles versus the CRP concentration φ are plotted as the dotted line curve in Fig. 4. The mathematical formula of OD-φ curve (the dotted line curve) can be expressed as: OD = 2.57 x 10⁻⁴ φ - 0.026 for φ ≥ 100 ng/ml. In general, the CRP solution in concentrations below 100 ng/ml will not be detectable via the traditional ELISA assay, since the sensitivity using the ELISA assay for human CRP is 100 ng/ml. Contrarily, through the magnetic labelling immunoassay, human CRP can be detected at a much lower concentration (e.g. as low as 10 ng/ml) using the magnetic nanoparticles. The detectable sensitivity of the magnetic labelling immunoassay is higher than that of the traditional ELISA assay by one order of magnitude in concentration.

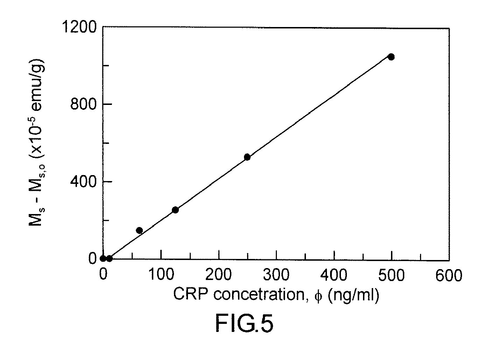

The solid line curve in Fig. 4 shows a residual M_{s,o} for a CRP concentration of zero. The non-zero residual Mₛ may be resultant from free magnetic nanoparticles that have diameters larger than the micro-holes of the filter, stayed on the filter. In this case, in practice, it is suggested that the saturated magnetization of the control solution without CRP (the reference solution) is measured first to obtain M_{s,o}, followed by measuring the Mₛ of the control solution with unknown concentrations of CRP to obtain the measured value of Mₛ at a given φ subtracted by the residual M_{s,o} (i.e. Mₛ - M_{s,o}). Fig. 5 shows the saturated magnetization difference (Mₛ - M_{s,o}) of the magnetic nanoparticles with CRP immune complexes as a function of CRP concentrations φ. After establishing the reference curve shown in Fig. 5, the CRP concentration of the sample solution can be determined.

Alternatively, the amount of the ligand in the sample can be measured based on the changes in the alternating current (ac) magnetic susceptibility reduction.

As discussed above, the added ligands may cause the agglomeration of the magnetic nanoparticles and result in the formation of magnetic particle clusters. On the other hand, if the magnetic fluid added with the ligands is not filtered by the micro-filter, magnetic particle clusters along with free magnetic nanoparticles co-exist in the solution when the added ligands are not in excess. When an external magnetic field is applied, the magnetic moments of single free nanoparticles and particle clusters are aligned along the external magnetic field. As the magnetic field is quenched, the single magnetic nanoparticles and particle clusters will relax with different relaxation behaviours. According to the reported data, the single magnetic nanoparticles show Brownian relaxation with a relaxation time constant of several microseconds, while the magnetic particle clusters exhibit Néel relaxation with a relaxation time constant of hundreds of milliseconds. Thus, under an external alternating current (ac) magnetic field with a frequency of several tens to 10⁶ Hz, only the magnetic moments of single magnetic nanoparticles are able to oscillate with the external ac magnetic field, while the magnetic moments of the particle clusters are almost held still. Hence, the ac magnetic susceptibility χ_{ac} of the solution is substantially attributed from the single magnetic nanoparticles, instead of the particle clusters. Therefore, the ac magnetic susceptibility χ_{ac} of the solution (magnetic fluid containing free magnetic nanoparticles) should become smaller after the addition of the ligands. This is because more particle clusters are formed and less free magnetic nanoparticles exist in the solution. As a result, the amount of the ligands can be measured based on the reductions in the values of the ac magnetic susceptibility χ_{ac}. That is, by measuring the ac magnetic susceptibility reduction (Δχ_{ac}) between the χ_{ac} of the solutions with and without ligands, the concentrations of the ligands in the sample can be determined. Similarly, before using the magnetic fluid to measure the sample including unknown amounts of the ligands, it is necessary to establish the relationship between the amount of the ligands and the ac magnetic susceptibility reduction (△χ_{ac}) from the control solution, by adding various amounts of the ligand in the control solution that includes magnetic nanoparticles coated with a fixed amount of the bio-receptor and obtaining the Δχ_{ac} by measuring the χ_{ac} before and after adding the ligand to the control solution.

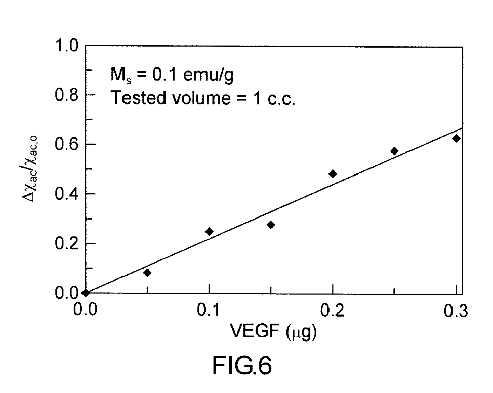

*Immunomagnetic detection of VEGF* Using the anti-VEGF antibody-VEGF pair (obtained from Biosource, Inc.) as an example, a linear relationship is obtained between the normalized ac magnetic susceptibility reductions Δχ_{ac}/χ_{ac,o} and the amounts of VEGF from zero to about 0.3 µg/ml, as shown in Fig. 6. It is noted in Fig. 6 that the data of VEGF-amount dependent Δχ_{ac}χ_{ac,o} fall in the same curve for variously concentrated (Mₛ) magnetic fluids having magnetic nanoparticles coated with anti-VEGF. This indicates that VEGF can be magnetically marked and quantitatively detected by the magnetic labelling immunoassay of this invention. This curve shown in Fig. 6 can be used as a reference curve to determine the amounts of VEGF using bio-functionalized magnetic nanoparticles through the measurement of △χ_{ac}/χ_{ac,o}.

Clearly, because the principles of the measuring methods proposed in this invention are based on magnetic properties of the magnetic fluid and/or the formed particle clusters, no fluorescence labels or coloring agents are required for determining the amount of the biomolecules or ligands in the sample. Hence, no extra processing steps and less human labor are needed and the costs of the test assays can be reduced.

Through the usage of the bio-functionalized magnetic nanoparticles, the magnetic labelling immunoassay provides both high specificity and excellent sensitivity toward the ligands or biomolecules to be detected or marked.

It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims and their equivalents.

## Claims

1. A diagnostic method using a magnetic labelling immunoassay for *in-vitro* quantitatively measuring an amount of ligands in a sample solution, the method comprising:
providing the sample solution containing the ligands;
applying the magnetic labelling immunoassay to the sample solution, wherein the magnetic labelling immunoassay comprises:
magnetic nanoparticles;
hydrophilic surfactants; and
bio-receptors, bound to the magnetic nanoparticles, wherein the bio-receptors are able to conjugate with the ligands so that the nanoparticles aggregate to form particle clusters;
filtrating the sample solution to obtain the particle clusters; and
measuring a saturated magnetization of the particle clusters to determine the amount of the ligands.

2. The method according to claim 1, further comprising establishing a relationship between the saturated magnetization of the particle clusters and amounts of the ligands by adding various amounts of the ligands to a control solution and measuring the saturated magnetization for the formed particle clusters in the control solution after the addition of the ligands, and wherein the amount of the ligands in the sample solution is determined based on the established relationship.

3. The method according to claim 1, further comprising establishing a relationship between a variation of the saturated magnetization of the particle clusters and amounts of the ligands by adding various amounts of the ligands to a control solution and measuring the difference in the saturated magnetization for the formed particle clusters in the control solution having the ligands from residual saturated magnetization of the control solution before adding the ligands to the control solution, and wherein the amount of the ligands in the sample solution is determined based on the established relationship.

4. The method according to claim 1, wherein the ligand is selected from the group consisting of vascular cell adhesion molecule-1 (VCAM-1), matrix metalloproteinase (MMP), intracellular adhesion molecule-1 (ICAM-1), vascular endothelial growth factor (VEGF), C-reactive protein (CRP), high-sensitivity CRP (hsCRP) and pigment epithelium-derived factor (PEDF).

5. The method according to claim 1, wherein the magnetic nanoparticles are Fe₂O₃ magnetic nanoparticles or Fe₃O₄ magnetic nanoparticles.

6. The method according to claim 1, wherein the magnetic nanoparticles are MnFe₂O₄ magnetic nanoparticles, NiFe₂O₄ magnetic nanoparticles, or CoFe₂O₄ magnetic nanoparticles.

7. A diagnostic method using a magnetic labelling immunoassay for *in-vitro* quantitatively measuring an amount of ligands in a sample solution, the method comprising:
providing the sample solution containing the ligands;
applying the magnetic labelling immunoassay to the sample solution, wherein the magnetic labelling immunoassay comprises:
magnetic nanoparticles in a solution;
hydrophilic surfactants; and
bio-receptors, bound to the magnetic nanoparticles, wherein the bio-receptors are able to conjugate with the ligands; and
measuring an ac magnetic susceptibility reduction of the sample solution to determine the amount of the ligands.

8. The method according to claim 7, further comprising establishing a relationship between the ac magnetic susceptibility reductions and amounts of the ligands by adding various amounts of the ligands to a control solution and measuring the ac magnetic susceptibility reductions of the control solution, and wherein the amount of the ligands in the sample solution is determined based on the established relationship.

9. The method according to claim 7, further comprising establishing a relationship between a normalized ac magnetic susceptibility reduction and amounts of the ligands by adding various amounts of ligands to a control solution, measuring the ac magnetic susceptibility reduction of the control solution and normalizing the ac magnetic susceptibility reduction of the control solution, and wherein the amount of the ligands in the sample solution is determined based on the established relationship.

10. The method according to claim 7, wherein a frequency range for the ac magnetic susceptibility is from several tens to 10⁶ Hz.

11. The methods according to claim 7, wherein the ligand is selected from the group consisting of vascular cell adhesion molecule-1 (VCAM-1), matrix metalloproteinase (MMP), intracellular adhesion molecule-1 (ICAM-1), vascular endothelial growth factor (VEGF), C-reactive protein (CRP), pigment epithelium-derived factor (PEDF) and high-sensitivity C-reactive protein (hsCRP).

12. The methods according to claim 7, wherein the magnetic nanoparticles are Fe₃O₄ magnetic nanoparticles, Fe₂O₃ magnetic nanoparticles, MnFe₂O₄ magnetic nanoparticles, NiFe₂O₄ magnetic nanoparticles, or CoFe₂O₄ magnetic nanoparticles.

13. A magnetic labelling immunoassay to detect ligands in a sample for assessing/evaluating statuses or risks of diseases, comprising:
magnetic nanoparticles, hydrophilic surfactants coated on surfaces of the magnetic nanoparticles; and bio-receptors bound to the hydrophilic surfactants on the magnetic nanoparticles, wherein the bio-receptors are able to conjugate with the ligands and the bio-receptors are selected from the group consisting of streptavidin-biotinylated antibodies for vascular cell adhesion molecule-1 (VCAM-1), matrix metalloproteinase (MMP), intracellular adhesion molecule-1 (ICAM-1), vascular endothelial growth factor (VEGF) and pigment epithelium-derived factor (PEDF), and antibodies for C-reactive protein (CRP) and high-sensitivity CRP (hsCRP).
